**Europäisches Patentamt**

**European Patent Office**

**Office européen des brevets**

(19)

(11) Veröffentlichungsnummer: **0 363 502 B1**

# (12) EUROPÄISCHE PATENTSCHRIFT

(45) Veröffentlichungstag der Patentschrift:
**25.09.91 Patentblatt 91/39**

(51) Int. Cl.⁵: **A61K 33/24, A61K 47/02, A61K 9/08, A61K 31/29**

(21) Anmeldenummer: **88116730.8**

(22) Anmeldetag: **08.10.88**

(54) **Flüssiges, wismuthaltiges Arzneipräparat, Verfahren zum Herstellen desselben und seine Verwendung.**

Die Akte enthält technische Angaben, die nach dem Eingang der Anmeldung eingereicht wurden und die nicht in dieser Patentschrift enthalten sind.

(43) Veröffentlichungstag der Anmeldung:
**18.04.90 Patentblatt 90/16**

(45) Bekanntmachung des Hinweises auf die Patenterteilung:
**25.09.91 Patentblatt 91/39**

(84) Benannte Vertragsstaaten:
**AT BE CH DE ES FR GB GR IT LI NL SE**

(56) Entgegenhaltungen:
**EP-A- 0 206 626
DE-A- 2 501 787
Gist-Brocades Unterlagen: Seiten 51-75 der Akte**

(73) Patentinhaber: **DR. R. PFLEGER CHEMISCHE FABRIK GMBH
Emil-Kemmer-Strasse 33
W-8605 Hallstadt (DE)**

(72) Erfinder: **Bertholdt, Heinz, Dr.
Birkenstrasse 3
W-8608 Memmelsdorf (DE)**
Erfinder: **Michalczyk, Dieter
Baumfeldstrasse 17
W-8608 Drosendorf (DE)**
Erfinder: **Urban, Günter
Rotdornstrasse 9
W-8605 Hallstadt (DE)**

(74) Vertreter: **Fleck, Thomas, Dr.Dipl.-Chem. et al
Patentanwälte Raffay, Fleck & Partner
Postfach 32 32 17
W-2000 Hamburg 13 (DE)**

EP 0 363 502 B1

## Beschreibung

Die Erfindung betrifft ein flüssiges, wismuthaltiges Arzneipräparat nach dem Oberbegriff des Anspruchs 1, ein Verfahren zum Herstellen desselben und seine Verwendung.

Flüssige Präparate, die Wismutsalze enthalten, sind generell bekannt ; vgl. DE-OS 1900268 oder EP-A-0217440. Da die als Lösungen oder Suspension bekannten pharmazeutischen Wismut-Präparate in offenen Systemen instabil sind, wurde schon früher ein geschlossenes System in zugeschmolzenen Ampullen eingesetzt, die jedoch wegen ihrer Nachteile vom Markt genommen worden sind. So wird in der letztgenannten Druckschrift die Instabilität wässriger Suspensionen erwähnt, wenn diese unterschiedlichen Bedingungen unterliegen. In der DE-PS 2501787 wird hervorgehoben, daß flüssige Präparate den Nachteil haben, daß sie weniger leicht zu lagern und zu transportieren sind als feste Präparate. Dort wird ferner erwähnt, so viel Ammoniak und Alkalimetallhydroxid zu verwenden, daß ein pH-Wert von 9 erreicht wird, insbesondere wenn die Flüssigkeit vor der Sprühtrocknung einige Zeit zu lagern ist. Stabile Lösungen lassen sich nur mit überschüssiger AmmoniakLösung herstellen, die jedoch wegen des starken Ammoniak-Geruchs patientenseitig keine Akzeptanz finden. Aus solchen stabilisierten Lösungen läßt sich jedoch durch Sprühtrocknung eine stabile Form des kolloiden Trikaliumwismutdicitrat-Komplexes gewinnen, die sich zur Herstellung von festen Arzneiformen, wie Film- oder Kautabletten, oder durch Wiederauflösen zur Herstellung von flüssigen, jedoch nur begrenzte Zeit haltbaren Arzneiformen verwenden läßt, vgl. hierzu die o.g. DE-PS 2501787.

Auch den in einer Arzneiflasche abgefüllten Trikaliumwismutdicitrat-Lösungen muß bekanntlich ein Ammoniaküberschuß vorhanden sein, der jedoch so zu dimensionieren ist, daß der bei der täglich mehrmals erforderlichen Entnahme der Lösung aus der Flasche zwangsläufig eintretende Ammoniak-Verlust die Stabilität der Lösung nicht gefährdet. Letzteres hat aber zur Folge, daß diese Lösung deutlich nach Ammoniak riecht und patientenseitig als störend empfunden wird.

Ferner hat sich gezeigt, daß bei wässerigen Lösungen der Zusatz von Konservierungsmitteln erforderlich ist, um das Arzneimittel ausreichend haltbar zu machen.

Ein weiterer Nachteil der in Arzneiflaschen angebotenen Lösungen liegt in der mangelhaften Dosiergenauigkeit, da meistens keine Dosierhilfe z.B. in Form eines Meßlöffels beigegeben wird, so daß irgendwelche bekanntlich sehr unterschiedlich dimensionierte Tee- oder Eßlöffel verwendet werden müssen.

Auch bei Tabletten ist die Dosierungsgenauigkeit der Einzel- oder Mehrfachdosis unbefriedigend, da diese in Abhängigkeit von dem Homogenitätsgrad des Pressgutes und vom Tablettengewicht schwankt.

Ein weiterer Nachteil bei Kautabletten liegt in ihrem langen Kontakt mit der Mundschleimhaut und der Dunkelfärbung von Zunge und Zahnschmelz. Auch muß sich der Wirkstoff erst im Speichel oder Magensaft lösen, um an den Wirkungsort der Magenschleimhaut gelangen zu können. Da der Wirkstoff erst nach Freisetzung aus der Tablette bzw. aus den beim Kauen entstehenden Tablettenpartikeln in Lösung gehen kann, steht er häufig lokal in unterschiedlichen Konzentrationen zur Verfügung. Da die Löslichkeit des Wirkstoffes auch pH-abhängig ist und die pH-Verhältnisse im Magen intraindividuellen und interindividuellen Schwankungen unterliegen, ist die Wirstofffreisetzung aus festen Zubereitungen und damit deren Wirkung nicht reproduzierbar. Schließlich sei noch erwähnt, daß sich auch bei Tabletten empfiehlt, Flüssigkeit nachzutrinken, um den Mundbereich von Resten einer Kautablette zu befreien. Es dürfte einleuchten, daß solche Tabletten bei Zahnprothesenträgern ohnehin nur eine geringe'Akzeptanz haben.

Der vorliegenden Erfindung liegt allgemein die Aufgabe zugrunde, die eben geschilderten Nachteile des Standes der Technik zu vermeiden und das eingangs genannte Arzneipräparat derart zu verbessern, daß es patientenfreundlich ist.

Ziel der Erfindung ist also ein solches Präparat, das nicht nur die medizinisch sinnvollste Applikation darstellt, sondern gleichzeitig geschmacks- und geruchsneutral ist und eine hohe Dosierungsgenauigkeit der Einzel- und Mehrfachdosis aufweist. Es dürfte einleuchten, daß das gesuchte Arzneipräparat ferner einfach und preiswert herzustellen sein und eine gute Lagerstabilität aufweisen sollte.

Diese Aufgabe wird einerseits durch das im Anspruch 1 gekennzeichnete Arzneipräparat bzw. andererseits durch das im Anspruch 7 gekennzeichnete Verfahren zu seiner Herstellung gelöst.

Überraschend konnte nämlich gezeigt werden, daß eine kolloide Lösung des Trikaliumwismutdicitrat-Komplexes aus Stabilitätsgründen Keinen Überschuß an Ammoniak erfordert, wenn die mit Ammoniak auf ca. pH 6 bis 7 eingestellte, geruchlose Lösung in ein geschlossenes System, z.B. in ein Eindosenbehältnis (z.B. Trinkampulle) oder in ein spezielles Mehrdosenbehältnis (z.B. Zweikammerdruckpackung mit Dosierventil), eingebracht wird. Die Lösung kann zudem noch sterilisiert werden und bedarf Keines Konservierungsmittelzusatzes, der bei einer Lösung im geschlossenen System für erforderlich gehalten wurde, und in einem offenen Mehrdosenbehältnis sogar unabdingbar ist.

Es versteht sich, daß mit der Erfindung eine problemlose Applikation möglich ist, da der Inhalt einer Trinkampulle dosisgerecht und problemlos aufgrund seiner Trinkfertigkeit einzunehmen ist. Die einem Mehrdosenbehältnis mit Dosiereinrichtung zu entnehmende Einzeldosis (z.B. 1-2 ml) kann mit Wasser beliebig verdünnt und dann ebenfalls problemlos eingenommen werden. Selbstverständlich kann das erfindungsgemäße Arzneipräparat zusätzlich mit Wasser oder anderen Flüssigkeiten beliebig verdünnt werden. Ebenfalls ist die flüssige Form des erfindungsgemäßen Arzneipräparates die medizinisch sinnvollste Applikation, da der Wirkstoff bereits gelöst angeboten wird und nach der Applikation für die Magenschleimhaut sofort optimal dispers verfügbar ist.

Darüberhinaus weist das erfindungsgemäße Arzneipräparat sowohl als Einzeldosenbehältnis bzw. als Mehrdosenbehältnis mit Dosiereinrichtung eine hohe Dosierungsgenauigkeit auf. Vorteilhafterweise enthält jede Einzeldosis eine Wirkstoffmenge entsprechend 120 mg $Bi_2O_3$. Ein Mehrdosenbehältnis enthält ein Vielfaches davon. Jegliche externen Dosierhilfen sind hierbei überflüssig.

Ebenso erweist sich das erfindungsgemäße Herstellverfahren des flüssigen wismuthaltigen Arzneipräparates als besonders einfach und wirtschaftlich, da keine komplizierte Sprühtrocknung — wie sie bei der Herstellung entsprechender Tabletten erforderlich ist — eingesetzt werden muß. Die einzelnen Verfahrensschritte a bis g sind im Anspruch 7 angegeben.

Auch läßt sich die Erfindung als Verwendung einer flüssigen, wismutammoniumcitrathaltigen Zubereitung zur Herstellung eines Arzneipräparates in Form einer stabilen, klaren, geruchs- und geschmacksneutralen Lösung mit einem pH-Wert von 6,0-7,0 in einem geschlossenen Ein- oder Mehrdosen- behältnis zur Behandlung campylobacter- bedingter Magen-Darm-Erkrankungen darstellen. Dabei ist durch die besondere Art des Behältnisses eine Kontamination durch Fremdkeime und eine Veränderung des Arzneipäparates (Ammoniakverlust) ausgeschlossen, so daß Stabilität, Haltbarkeit und Verwendbarkeit des Präparates nicht negativ beeinflußt werden.

Weitere Vorteile und Merkmale gehen aus den Unteransprüchen hervor. Im folgenden wird zum besseren Verständnis der Erfindung ein bevorzugtes Ausführungsbeispiel anhand einer Rezeptur beschrieben :

100 mMol Wismutcitrat werden in Wasser suspendiert und in einem geschlossenen System mit einer wässerigen Lösung von 70 mMol Kaliumhydroxid und 115 mMol Ammoniak versetzt. Nach Zusatz einer wässerigen Lösung von 35 mMol Trikaliumcitrat und ca. 2 mMol Zitronensäure wird mit Wasser auf ein Endvolumen von 390 ml ergänzt. Die klare Lösung wird entweder unverdünnt in dem geschlossenen System durch Erhitzen sterilisiert und anschließend in Mehrdosenbehältnisse mit Dosiereinrichtung eingebracht oder im Verhältnis 1 : 5 mit Wasser verdünnt und dann in Eindosenbehältnisse eingebracht, wobei die verdünnte Lösung, je nach Materialbeschaffenheit des Behältnisses, vor oder nach dem Einbringen in die Eindosenbehältnisse sterilisiert wird.

## Patentansprüche

1. Flüssiges, wismuthaltiges Arzneipräparat zur oralen Behandlung campylobacter-bedingter Magen-Darm-Erkrankungen, wie Gastritis oder Ulcus in Form eines geschlossenen Systems, gekennzeichnet durch eine stabile, klare, geruchs- und geschmacksneutrale Lösung von Wismut (III)-citrat-hydroxid-Komplex, Ammonium-Kalium-Salz mit einem pH-Wert von 6,0-7,0, die keinen Zusatz von Konservierungsmitteln enthält.

2. Flüssiges, wismuthaltiges Arzneipräparat nach Anspruch 1, gekennzeichnet durch ein Ein- oder Mehrdosenbehältnis mit Dosiereinrichtung (z.B. Zweikammerdruckpackung mit Dosiervorrichtung), das eine Ein- bzw. Mehrfachdosis des wismuthaltigen Arzneipräparates enthält.

3. Flüssiges, wismuthaltiges Arzneipräparat nach einem oder mehreren der vorstehenden Ansprüche, dadurch gekennzeichnet, daß das Behältnis aus Glas, Kunststoff, Metall oder deren Kombination besteht.

4. Flüssiges, wismuthaltiges Arzneipräparat nach Anspruch 1, dadurch gekennzeichnet, daß Einzeldosenbehältnisse ein Wirkstoffäquivalent von 100 bis 140 mg $Bi_2O_3$, insbesondere 120 mg $Bi_2O_3$ in 5-15 ml, vorzugsweise 10 ml enthalten.

5. Flüssiges, wismuthaltiges Arzneipräparat nach einem oder mehreren der vorstehenden Ansprüche, dadurch gekennzeichnet, daß Mehrdosenbehältnisse ein Vielfaches des Wirkstoffäquivalentes von 100 bis 140 mg $Bi_2O_3$, vorzugsweise 120 mg $Bi_2O_3$ in 1-5 ml, vorzugsweise in 2 ml enthalten.

6. Flüssiges, wismuthaltiges Arzneipräparat nach einem oder mehreren der vorstehenden Ansprüche, dadurch gekennzeichnet, daß das geschlossene System folgende Bestandteile in Form einer stabilen, klaren, geruchs- und geschmacksneutralen Lösung umfaßt :

   a. Wismut-ammonium-citrat

   b. Trikaliumcitrat

   c. Wasser

   d. ggf. Zuschlagstoffe wie z.B. Aromastoffe, Farbstoffe, Stabilisatoren.

7. Verfahren zum Herstellen des flüssigen, wismuthaltigen Arzneipräparates nach Anspruch 1, gekennzeichnet durch folgende Schritte :

   a. Suspendieren von Wismutsubcitrat in Wasser;

   b. Erwärmen und Lösen der Suspension unter Zusatz wässeriger Kalilauge und Ammoniak-Lösung, wobei ein pH-Wert von 6,0-7,0, insbesondere 6, 5, eingestellt wird ;

   c. Zufügen einer wässerigen Trikaliumcitrat-Zitronensäure-Lösung ;

d. ggf. Zugabe weiterer Zuschlagstoffe ;

e. ggf. weiterer Zusatz von Wasser ;

f. ggf. Sterilisation der Lösung und Abfülien in Mehrdosenbehältnis mit Dosiereinrichtung ;

g. ggf. Abfüllen der Lösung in ein Eindosenbehältnis, Verschließen und Sterilisation desselben.

8. Verwendung einer flüssigen, wismuthaltigen Zubereitung zur Herstellung eines Arzneipräparates in Form einer stabilen, klaren, geruchs- und geschmacksneutralen Lösung von Wismut (III)-citrat-hydroxid-Komplex, Ammonium-Kalium-Salz mit einem pH-Wert von 6,0-7,0 in einem geschlossenen Ein- oder Mehrdosenbehältnis zur Behandlung campylobacter-bedingter Magen-Darm-Erkrankungen.

## Claims

1. Liquid, bismuth-containing medicinal product for oral treatment of gastro-intestinal illnesses, such as gastritus or ulcers, caused by campylobacter in form of a closed system, characterized by a stable clear solution neutral as regards odor and taste of bismuth (III)-citrate-hydroxide complex, ammonium-potassium salt with a pH-value of 6,0 to 7,0 comprising no addition of preservatives.

2. Liquid, bismuth-containing medicinal product according to claim 1, characterized in that the single or multidose container with dosing device (e.g. a two-chamber pressure pack with dosing device) containing a single or multiple dose of the bismuth-containing medicinal product.

3. Liquid, bismuth-containing medicinal product according to claim 1, characterized in that the container is made from glass, plastic, metal or combinations thereof.

4. Liquid, bismuth-containing medicinal product according to claim 1, characterized in that single-dose containers contain an active substance equivalent of 100 to 140 mg of $Bi_2O_3$, particularly 120 mg of $Bi_2O_3$ in 5 to 15 ml and preferably 10 ml.

5. Liquid, bismuth-containing medicinal product according to one or more of the preceeding claims, characterized in that the multidose container contains a multiple of the active substance equivalent of 100 to 140 mg of $Bi_2O_3$, preferably 120 mg of $Bi_2O_3$ in 1 to 5 ml and preferably in 2 ml.

6. Liquid, bismuth-containing medicinal product according to one or more of the preceeding claims, characterized in that the closed system comprises the following constituents in the form of a stable, clear solution neutral as regards odor and taste :

a. Bismuth-ammonium-citrate

b. Tricalcium citrate

c. Water

d. Optionally additives such as e.g. flavours, colouring agents and stabilizers.

7. Process for the production of the liquid, bismuth-containing medicinal product according to claim 1, characterized by the following steps :

a. Suspending bismuth subcitrate in water,

b. Heating and dissolving the suspension, accompanied by the addition of aqueous caustic potash solution and ammonia solution, setting a pH-value of 6.0 to 7.0 and in particular 6.5,

c. Adding an aqueous triptoassium citrate-citric acid solution,

d. Optional addition of further additives

e. Optional addition of water,

f. Optional sterilization of the solution and filling into a multidose container with dosing device,

g. Optional filling of the ready-to-drink solution into a single-dose container, sealing and sterilization thereof.

8. Use of a liquid, bismuth-containing product for the preparation of a medical product in form of a stable, clear solution neutral as regards odor and taste of bismuth (III)-citrate-hydroxide complex, ammonium-potassium salt with a pH-value of 6,0 to 7,0 in a closed single or multidose container for treatment of gastro-intestinal illnesses caused by campylobacter.

## Revendications

1. Préparation médicamenteuse liquide, contenant du bismuth, pour le traitement par voie orale d'affections gastro-intestinales à campylobacter telles que la gastrite ou l'ulcère sous la forme d'un système fermé, caractérisée par une solution stable, limpide, inodore et insipide de complexe citrate-hydroxyde de bismuth (III), sel d'ammoniumpotassium, ayant un pH de 6,0 à 7,0, ne contenant pas d'addition de conservateurs.

2. Préparation médicamenteuse liquide, contenant du bismuth selon la revendication 1, caractérisée par un récipient à une ou plusieurs doses avec dispositif de dosage (par exemple un emballage sous pression à deux chambres avec dispositif de dosage), qui contient une dose unique ou multiple de la préparation médicamenteuse contenant du bismuth.

3. Préparation médicamenteuse liquide, contenant du bismuth, selon l'une ou plusieurs des revendications précédentes, caractérisée en ce que le récipient est constitué de verre, de matière plastique, de métal ou d'une association de ceux-ci.

4. Préparation médicamenteuse liquide, contenant du bismuth selon la revendication 1, caractérisée en ce que les récipients pour doses unitaires contiennent un équivalent en principe actif de 100 à 140 mg de $Bi_2O_3$, de préférence 120 mg de $Bi_2O_3$ dans 5-15 ml, de préférence dans 10 ml.

5. Préparation médicamenteuse liquide, contenant du bismuth, selon l'une ou plusieurs des revendications précédentes, caractérisée en ce que les récipients pour doses multiples contiennent un multiple de l'équivalent de principe actif de 100 à 140 mg de $Bi_2O_3$, de préférence de 120 mg de $Bi_2O_3$ dans 1 à 5 ml, de préférence dans 2 ml.

6. Préparation médicamenteuse liquide, contenant du bismuth, selon l'une ou plusieurs des revendications précédentes, caractérisée en ce que le système fermé comprend les constituants suivants sous la forme d'une solution stable, limpide, inodore et insipide de :

    a) citrate de bismuth et d'ammonium

    b) citrate tricalcique

    c) eau

    d) le cas échéant, des additifs tels que par exemple des substances aromatiques, des colorants, des stabilisants.

7. Procédé d'obtention de la préparation médicamenteuse liquide, contenant du bismuth, selon la revendication 1, caractérisé par les étapes suivantes:

    a) mise en suspension du sous-citrate de bismuth dans de l'eau ;

    b) chauffage et dissolution de la suspension par addition de lessive de potasse aqueuse et d'ammoniaque, en ajustant à un pH de 6,0-7,0, en particulier de 6, 5 ;

    c) addition d'une solution aqueuse de citrate tripotassique-acide citrique ;

    d) le cas échéant, addition d'autres additifs ;

    e) le cas échéant, addition supplémentaire d'eau;

    f) le cas échéant, stérilisation de la solution et introduction de celle-ci dans un récipient à doses multiples avec dispositif de dosage ;

    g) le cas échéant, introduction de la solution dans un récipient à dose unique, fermeture et stérilisation de celui-ci.

8. Utilisation d'une préparation liquide, contenant du bismuth, pour l'obtention d'une préparation médicamenteuse sous la forme d'une solution stable limpide, inodore et insipide de complexe citrate-hydroxyde de bismuth (III), sel d'ammonium et de potassium, ayant un pH de 6,0-7,0 dans un récipient fermé à une ou plusieurs doses pour le traitement des affections gastro-intestinales à campylobacter.